# EUROPEAN PATENT APPLICATION

(11) **EP 2 749 226 A1**
(43) Date of publication of application: **02.07.2014**
(21) Application number: 13196354.8
(22) Date of filing: 10.12.2013
(51) Int. Cl.: A61B 8/00, A61B 5/00, A61B 8/08, G06T 7/00

(54) **Object information acquiring apparatus**

(30) Priority: 28.12.2012 JP 2012286548
(71) Applicant: Canon Kabushiki Kaisha, Tokyo 146-8501 (JP)
(72) Inventor: Sato, Akira, Tokyo, Tokyo 146-8501 (JP); Abe, Hiroshi, Tokyo, Tokyo 146-8501 (JP)
(74) Representative: TBK

(57) **Abstract**

Provided is an object information acquiring apparatus including a light source, a photoacoustic probe that receives a photoacoustic wave generated at an object irradiated with light, an ultrasound probe that receives an ultrasound wave transmitted to the object and then reflected within the object, and a signal processor that creates a functional image showing functional information within the object on the basis of the photoacoustic wave and creates a morphological image showing morphological information within the object on the basis of the ultrasound wave, the signal processor being configured to divide the functional image into a plurality of regions, perform different image processing for each of the regions, superimpose the processed functional image on the morphological image, and display the resulting image in a display.

## Description

### BACKGROUND OF THE INVENTION

### Field of the Invention

The present invention relates to an object information acquiring apparatus.

### Description of the Related Art

In recent years, a technique has been devised in which an ultrasound wave (photoacoustic wave) from a photoacoustic effect is generated through irradiation of a living body with laser light and the photoacoustic wave is analyzed. This technique can image the structure and condition on the surface and the inside of the living body (see U.S. Patent Specification No. 5,840,023). This technique is called photoacoustic measurement or photoacoustic tomography. Since examination can be performed in a non-invasive manner, there are trends for medical application in order to examine the inside of a human body. For example, a photoacoustic measurement apparatus aimed at breast cancer screening has been developed (see S.A. Ermilov et al., "Development of laser optoacoustic and ultrasonic imaging system for breast cancer utilizing handheld array probes", Photons Plus Ultrasound: Imaging and Sensing 2009, Proc. of SPIE vol. 7177, 2009).
Patent Literature 1: U.S. Patent Specification No. 5,840,023
Non Patent Literature 1: S. A. Ermilov et al. , Development of laser optoacoustic and ultrasonic imaging system for breast cancer utilizing handheld array probes, Photons Plus Ultrasound: Imaging and Sensing 2009, Proc. of SPIE vol. 7177, 2009.

### SUMMARY OF THE INVENTION

A photoacoustic measurement apparatus can acquire various information including an optical characteristic value such as the light absorption coefficient within an object, depending on the wavelength of irradiation light for the object. For example, when near-infrared light having properties of being easily absorbed by hemoglobin within blood is used, a blood vessel image can be acquired.

By performing measurement a plurality of times using irradiation light of a wavelength easily absorbed by oxyhemoglobin and irradiation light of a wavelength easily absorbed by deoxyhemoglobin and comparing a plurality of acquired blood vessel images, the oxygen saturation within blood can be measured. An image showing the light absorption coefficient distribution or an image showing the oxygen saturation distribution is a functional image showing the functional distribution within an object.

According to S.A. Ermilov et al., "Development of laser optoacoustic and ultrasonic imaging system for breast cancer utilizing handheld array probes", Photons Plus Ultrasound: Imaging and Sensing 2009, Proc. of SPIE vol. 7177, 2009, an echo image of the inside of an object can be obtained by transmitting an ultrasound wave to the object and receiving an echo wave reflected inside the object. This is a morphological image showing the morphological structure within the object.

When a functional image obtained in photoacoustic measurement and a morphological image obtained in ultrasound wave measurement are superimposed, there are cases where comparing functional information and morphological information is difficult.

The present invention in its first aspect provides an object information acquiring apparatus as specified in claim 1 to 10.

Further features of the present invention will become apparent from the following description of exemplary embodiments with reference to the attached drawings.

### BRIEF DESCRIPTION OF THE DRAWINGS

Fig. 1 is an overall configuration diagram of an object information acquiring apparatus of the present invention;
Fig. 2 is a flowchart of the entire processing in the present invention;
Fig. 3 is a diagram showing one example of a GUI that performs superimposed image adjustment processing in the present invention;
Fig. 4 is a flowchart of superimposed image processing in Example 1; and
Figs. 5A to 5E are illustrations of superimposed image processing in Example 2.

### DESCRIPTION OF THE EMBODIMENTS

As described above, when a functional image obtained in photoacoustic measurement and a morphological image obtained in ultrasound wave measurement are superimposed, there are cases where comparing functional information and morphological information is difficult. To give a specific example, there is a case where a tumor area, a contour area of a tissue, or the like within a morphological image is hidden when a light absorption coefficient distribution image (functional image) of the inside of a breast is superimposed on an ultrasound tomographic image (morphological image) of the breast. Although dependent on the wavelength of irradiation light, the light absorption coefficient distribution obtained in photoacoustic measurement of a breast is derived mainly from a photoacoustic wave upon light absorption by hemoglobin. Therefore, a functional image mainly shows a blood vessel image. However, an acoustic wave, although weak, can be generated through light absorption in a portion other than a blood vessel. Thus, when a functional image is used directly as an image for superimposition, there have been cases where a portion unnecessary for diagnosis (portion other than a blood vessel) is superimposed on a morphological image to decrease the visibility. Even in cases where only a blood vessel of a certain scale or greater is under attention upon diagnosis, there have been cases where a blood vessel image of a small scale is displayed in a superimposed image.

The present invention has been made in view of the task described above and is developed to make comparison of functional information and morphological information easy upon superimposition and display of a functional image obtained by photoacoustic measurement and a morphological image obtained by ultrasound wave measurement.

A preferred embodiment of the present invention will be described below with reference to the drawings. Note that the dimension, material, and shape of components, the relative arrangement thereof, and the like described below should be changed appropriately depending on the configuration of an apparatus or various conditions to which the invention is applied and are not intended to limit the scope of this invention to the description below. The same components are denoted by the same reference numeral in principle, and description is omitted.

### (Apparatus configuration)

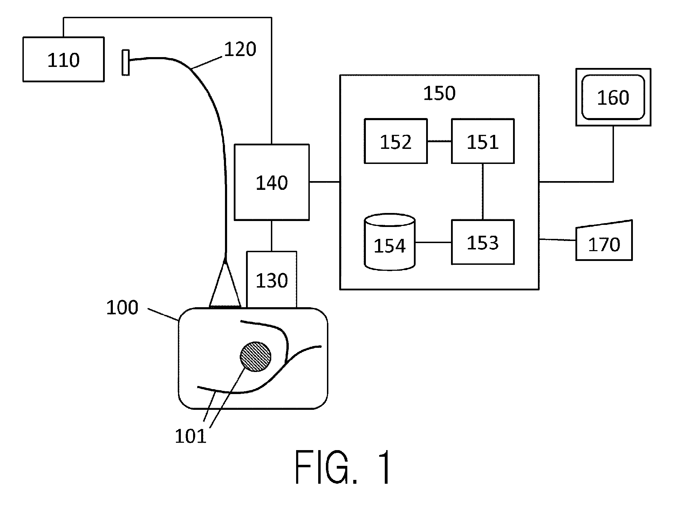

First, an object information acquiring apparatus according to this embodiment will be described using Fig. 1. The object information acquiring apparatus shown in Fig. 1 includes, as main components, a light source 110, an optical system 120, an acoustic wave detector 130, a control device 140, a signal processing device 150, a display device 160, and an input device 170.

The acoustic wave detector 130 of this embodiment may have both a function of an ultrasound wave transmitter that transmits an ultrasound wave to an object 100 and a function of an ultrasound wave receiver that detects an ultrasound wave propagated inside the object 100. The functions of transmission and reception may be performed by separate mechanisms. The reception of a reflected wave and the reception of a photoacoustic wave may be performed by separate mechanisms in accordance with the wavelength. In this case, an acoustic wave detector for reflected wave reception corresponds to an ultrasound probe of the present invention, and an acoustic wave detector for photoacoustic wave reception to a photoacoustic probe of the present invention. The control device 140 and the signal processing device 150 may be configured integrally. Hereinafter, each configuration will be described.

### (Object 100 and light absorber 101)

These are not part of the configuration of the object information acquiring apparatus of the present invention, but will be described below. The object information acquiring apparatus of the present invention is mainly aimed at diagnosis of malignant tumor, blood vessel disease, or the like of a human or animal, a follow-up of chemical treatment, or the like. Thus, as an object, a living body, specifically, a target segment for a diagnosis such as a breast, neck, or stomach of a human body or animal is assumed. Note that a phantom or the like may also be a target of measurement.

A light absorber is a portion inside an object and relatively high in the absorption coefficient. For example, if a human body is the target of measurement, oxyhemoglobin or deoxyhemoglobin, a blood vessel high in the same, or malignant tumor including a lot of new blood vessels is a light absorber. Plaque of a carotid wall is also a light absorber.

### (Light source 110)

As the light source, a pulsed light source capable of generating pulsed light in the order of several nanoseconds to several microseconds is preferable. Specifically, in order to generate a photoacoustic wave efficiently, a pulse width of approximately ten nanoseconds is used. As the light source, a light-emitting diode or the like may be used instead of a laser. As the laser, various lasers such as a solid-state laser, gas laser, dye laser, or semiconductor laser may be used.

Although an example of a single light source is shown in the drawing, a plurality of light sources may be used. In the case of a plurality of light sources, a plurality of light sources that causes oscillation in the same wavelengths may be used in order to increase the irradiation intensity of light with which a living body is irradiated, or a plurality of light sources with different oscillation wavelengths may be used in order to measure the difference in the optical characteristic value distribution depending on the wavelength.

For light irradiation in a plurality of wavelengths, a pigment capable of converting the oscillation wavelength or an optical parameter oscillator (OPO) may be used as the light source. Regarding the wavelength to be used, a region of 700 nm to 1100 nm where in vivo absorption is little is preferable. Note that, in the case of obtaining the optical characteristic value distribution of a living tissue relatively near the surface of a living body, a wavelength region of, for example, 400 nm to 1600 nm that is a range greater than a wavelength region described above may be used.

### (Optical system 120)

Light emitted from the light source is guided to the object while being processed into a desired light distribution shape with an optical part. Light may be propagated using an optical waveguide such as an optical fiber. The optical part is, for example, a mirror that reflects light, a lens that collects, magnifies, or changes the shape of light, or a diffuser that diffuses light. Any such optical part may be used, as long as the object is irradiated with light emitted from the light source in a desired shape. Light is preferably spread in an area of a certain degree rather than being collected with a lens, in terms of safety of a living body and increasing the diagnosis area.

### (Acoustic wave detector 130)

With the acoustic wave detector, a photoacoustic wave is received by a detection element and converted to an electrical signal that is an analog signal. Any detector such as those using a piezoelectric phenomenon, resonance of light, change in capacitance, or the like may be used, as long as an acoustic wave signal can be detected. The acoustic wave detector is preferably one in which a plurality of detection elements are arranged on an array, in terms of increasing measurement efficiency and improving the precision.

It is desirable that the acoustic wave detector have not only a function of receiving but also transmitting an ultrasound wave (acoustic wave), for the reason of signal detection in the same region and saving space. Although the bandwidth of an ultrasound wave received in ultrasound wave measurement and the bandwidth of a photoacoustic wave received in photoacoustic measurement do not necessarily match, it is preferable to employ a wide range detector that covers both bandwidths for the same reason.

An acoustic wave referred to in the present invention is typically an ultrasound wave and includes an elastic wave called a sound wave, ultrasound wave, or acoustic wave. An acoustic wave generated by a photoacoustic effect is called a photoacoustic wave or light-induced ultrasound wave.

### (Control device 140)

The control device amplifies an electrical signal obtained by the acoustic wave detector, and converts the electrical signal from an analog signal to a digital signal. The control device is typically configured of an amplifier, an A/D converter, a field programmable gate array (FPGA) chip, and the like. In the case where a plurality of electrical signals are obtained from the acoustic wave detector, it is desirable that a plurality of signals can be processed simultaneously to shorten the time for image formation.

The control device 140 performs control of light-emission timing of pulsed light generated from the light source and control of transmission and reception of an electrical signal for which pulsed light is a trigger signal. By performing addition processing after delay addition processing for phase matching of signals is performed, the FPGA chip can form a characteristic distribution of acoustic impedance or the like of an object or data of a speckle pattern caused by scattering within an object.

### (Signal processing device 150)

A workstation or the like is used as the signal processing device. Correction processing, image reconstruction processing, or the like is performed by software programmed in advance. For example, software used in a workstation includes superimposed image creation module 151 that performs superimposition processing that is processing characteristic in the present invention. As other software, modules such as an image reconstruction module 152 and a threshold management module 153 are included. Each module may be provided as a device separate from the signal processing device 150. The signal processing device 150 can apply signal processing to either a two-dimensional space or a three-dimensional space. The signal processing device corresponds to a signal processor of the present invention.

Details of image processing performed by the superimposed image creation module 151 and a threshold managed by the threshold management module 153 differ depending on the example, and therefore will be mentioned in the description for each example.

The image reconstruction module 152 performs image reconstruction using an acoustic wave signal and forms a characteristic distribution of acoustic impedance or the like or optical characteristic value distribution of an object. As an image reconstruction algorithm, back projection with time domain or Fourier domain that is normally used in a tomography technique, or delay and sum is used, for example. In the case where much time is allowed for reconstruction, an inverse analysis method with repetition processing may be used.

By using a focused acoustic wave detector in photoacoustic imaging, an in vivo optical characteristic value distribution image can be formed without image reconstruction. In such a case, it is not necessary to perform signal processing using an image reconstruction algorithm.

There may be cases where the control device and the signal processing device are integrated. In this case, a characteristic distribution of acoustic impedance or the like or optical characteristic value distribution of an object may be created by hardware processing instead of software processing performed with a workstation.

### (Display device 160)

The display device is a device that displays an image output from the signal processing device. Typically, a liquid crystal display or the like is used. However, a display in other forms such as a plasma display, organic EL display, or FED is acceptable. A display may be provided separately from the object information acquiring apparatus of the present invention. The display device corresponds to a display of the present invention.

### (Input device 170)

The input device is a mechanism that accepts setting indication of a predetermined threshold parameter by a user or setting change while looking at a display. A button, mouse, voice, or anything else is acceptable, as long as a user can set a numerical value or the like in correspondence with what is displayed on a display. The input device corresponds to an input unit of the present invention.

### (Procedure of entire processing)

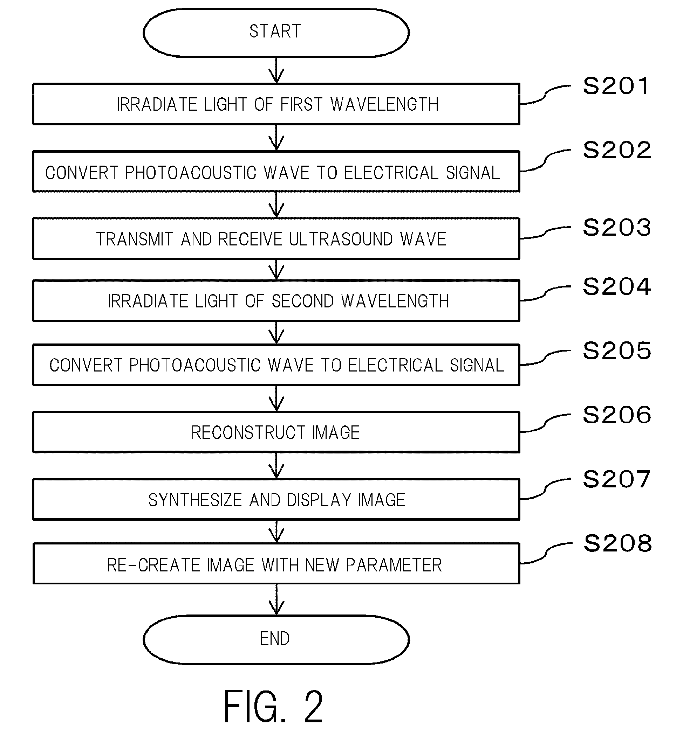

Using a flowchart in Fig. 2, a typical procedure of object information acquisition by an apparatus with the configuration described above will be described.

The control device 140 starts acoustic measurement control in accordance with a measurement indication by a user.

In step S201, the light source 110 radiates light of a first wavelength. The irradiation light is guided to the surface of the object 100 via the optical system 120. At this time, beam forming is performed in the optical system 120, and the irradiation range, light intensity distribution, or the like of the irradiation light is arranged. The irradiation light propagates inside the object 100, and a photoacoustic wave from the first wavelength is generated from the light absorber 101.

In step S202, a first photoacoustic wave is received and converted to an electrical signal by the acoustic wave detector 130. The electrical signal is transferred to the signal processing device 150 as a photoacoustic wave signal caused by the first wavelength via A/D conversion processing, and stored in an HDD 154 that is storage means.

At this time, in step S203, ultrasound wave measurement is performed by transmitting and receiving an ultrasound wave after a certain delay time with pulsed light generated from the light source as a trigger, as mentioned in the description of the control device 140. At this time, an ultrasound wave signal reflected and propagated from within a living body and acquired herein is received by the acoustic wave detector 130, converted to an electrical signal by the control device 140, then transferred to the signal processing device 150 as an ultrasound wave signal via A/D conversion processing, and stored in the HDD 154.

In steps S204 and S205, processing similar to steps S202 and S203 described above is performed. That is, the light source 110 irradiates the surface of the object 100 with light of a second wavelength, and a photoacoustic wave signal caused by the second wavelength is acquired and stored in the HDD 154.

Since a morphological image from ultrasound wave measurement is already acquired, ultrasound wave measurement does not necessarily need to be performed.

In step S206, the image reconstruction module 152 creates an image from the photoacoustic wave signal and the ultrasound wave signal acquired in the respective steps described above. Specifically, a functional image acquired with the first wavelength, a functional image acquired with the second wavelength, and the morphological image acquired with an ultrasound wave are reconstructed.

In step S207, the functional image for each wavelength is synthesized with the morphological image by the superimposed image creation module 151. At this time, the superimposed image creation module 151 acquires a parameter threshold for creating a superimposed image from the threshold management module 153, and processes functional information image to create a superimposition functional information image.

A functional image for superimposition is created in the following manner. The functional image is divided into a plurality of regions in accordance with a predetermined parameter threshold, and image processing is performed for each region depending on a desired image processing setting parameter prepared for each region. The image processing setting parameter is specifically the transparency (transmittance) or a hue setting. In the case of the transmittance, different transparent image processing is performed for each region in the functional image divided into the plurality of regions. In the case of the hue setting, different color conversion processing is performed for each region of the functional image as image processing to make unnecessary portions less conspicuous.

The superimposition functional information image created in this manner is superimposed through processing on the morphological image and displayed in the display device 160. At this time, a segment under attention of high importance determined in accordance with the threshold and other segments not under attention are distinguished by, for example, the transmittance, and the visibility of the segment under attention is increased.

In step S208, a user sets a new parameter threshold in the threshold management module 153 using the input device 170 while checking an image to re-create the superimposed image.

A photoacoustic image corresponding to each of the two wavelengths is superimposed on an ultrasound wave image in the flow described above. However, in reality, this is not limiting. As an original image with which a functional image for superimposition is created, a photoacoustic image based on a single wavelength may be used, or an image showing the oxygen saturation that is created from a measurement result of two wavelengths may be used.

A user may indicate a boundary for a region of a segment under attention and a segment not under attention using the input device 170 to divide a functional image into a plurality of regions. The image processing as described above can be performed with respect to each of the plurality of regions set in this manner.

### (User interface)

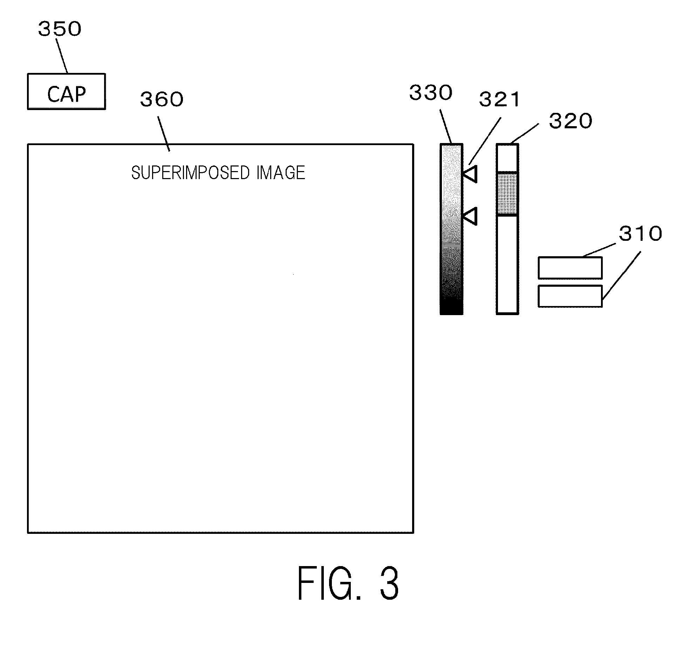

One example of a UI operated by a user is shown in Fig. 3.

A button 350 is an image acquisition trigger button (capture button). When this is pressed, photoacoustic wave measurement and ultrasound wave measurement are performed, and a superimposed image 360 is displayed. A color bar 330 is for checking the hue of display in each range of measurement values of a functional image.

A text box 310 is a text box for setting an upper limit setting value and a lower limit setting value as a threshold. The setting of threshold may be through means that enables intuitive operation such as a slider bar. As shown with reference numerals 320 and 321 in the drawing, preparing a display with which a set threshold range can be checked in comparison with an entire range makes checking of the state of setting easy. When a threshold is changed by a user, the superimposed image is re-processed on the basis of a setting threshold at a predetermined frequency, and the display is updated. A user can set a threshold while checking the superimposed image 360.

In the description below, a morphological image is acquired by ultrasound wave measurement and used for a superimposed image. However, information that is generally known to be obtained in ultrasound wave measurement such as blood flow information from Doppler analysis may be imaged and used.

### <Example 1>

Next, a preferred embodiment of an object information acquiring method using the object information acquiring apparatus shown in Fig. 1 will be described.

### (Case of application to light absorption coefficient distribution)

In the object information acquiring apparatus shown in Fig. 1, a functional image created through photoacoustic wave measurement shows the light absorption coefficient distribution. In this example, the wavelength of irradiation light is adjusted such that a photoacoustic effect occurs in hemoglobin. Therefore, an acquired photoacoustic signal is mainly a photoacoustic wave generated by hemoglobin. A strong signal is obtained from a blood vessel of a large scale, and a weak signal from a blood vessel of a small scale such as a capillary. This is imaged as a distribution image of the light absorption coefficient showing the light absorption rate.

In this example, a threshold parameter managed by the threshold management module 153 is the light absorption coefficient that is one of optical characteristic values. First, light absorption coefficient distribution data and an ultrasound wave image are acquired. The light absorption coefficient distribution data is a data string showing the light absorption coefficient distribution in a measurement region (flat region in this example) obtained by reconstruction processing with respect to a result of photoacoustic measurement. The ultrasound wave image is an ultrasound tomographic image in the same region as a region in which photoacoustic measurement is performed.

### (Superimposed image processing)

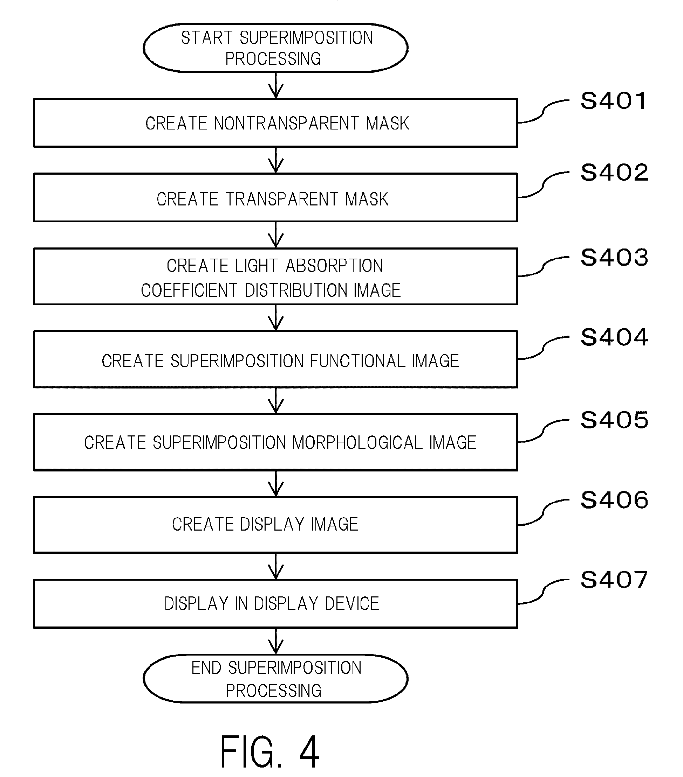

Hereinafter, superimposed image processing performed by the superimposed image creation module 151 will be described with reference to Fig. 4. First, the flow is started by the superimposed image creation module 151 acquiring the threshold parameter from the threshold management module 153.

In step S401, the superimposed image creation module 151 compares the light absorption coefficient distribution data and the threshold parameter, extracts a portion in which the value of the light absorption coefficient is greater than or equal to the threshold parameter, and creates nontransparent mask data. At this time, the nontransparent mask data is a binary data string in which a portion greater than or equal to a threshold is expressed as 1 and a portion less than a threshold as 0.

In step S402, the superimposed image creation module 151 extracts a portion in which the value of the light absorption coefficient is smaller than the threshold parameter and creates transparent mask data. Since the transparent mask data is an inverse image of the nontransparent mask data, it suffices to create inverted data of a nontransparent mask.

In step S403, the superimposed image creation module 151 creates a light absorption coefficient distribution image using the light absorption coefficient distribution data. In this example, the light absorption coefficient distribution image is a color image in which a different hue is allocated in accordance with the light absorption coefficient.

In step S404, the superimposed image creation module 151 applies the transparent mask data with respect to the light absorption coefficient distribution image created in S403 to create a superimposition functional image. For the superimposition functional image, transparency processing is performed with respect to the light absorption coefficient distribution image in a pixel position corresponding to 1 of the transparent mask data.

In step S405, the superimposed image creation module 151 applies the nontransparent mask data created in S401 with respect to the ultrasound tomographic image to create a superimposition morphological image. For the superimposition morphological image, transparency processing is performed with respect to the ultrasound tomographic image in a pixel position corresponding to 1 of the nontransparent mask data.

In step S406, the superimposed image creation module 151 performs superimposition processing of the superimposition functional image created in step S404 and the superimposition morphological image created in step S405 to create a display image. In step S407, the created image is displayed by the display device.

In the morphological image obtained by ultrasound wave measurement, portions differing in echo intensity inside the object are imaged, and a boundary between different densities of a living tissue is depicted. Particularly in an ultrasound wave image of the inside of a breast, a tumor area may appear as a region called a low echoic area that is black and dark. A boundary in a living tissue is important in checking the state of distribution and the position of a blood vessel inside a living body, and a low echoic area is important in checking the state of distribution of functional information in the periphery of the tumor area. Since a boundary and a low echoic area are both segments under attention in an ultrasound wave image, it is not preferable that these be covered and hidden.

Since a superimposition light absorption coefficient distribution image created in accordance with the flow described above is subjected to transparency processing for a segment not under attention, it is less likely that a low echoic area or a boundary of a living tissue is covered and hidden by the segment not under attention, even in the case of superimposition on a morphological image. As a result, a sense of inhibition due to unnecessary information is reduced upon checking the relevance of functional information of an affected area and morphological information near the affected area, and an image is made more visible.

### <Example 2>

### (Case of application to oxygen saturation)

A functional image created in this example is an oxygen saturation distribution image obtained by photoacoustic measurement with a plurality of wavelengths.

By comparing a plurality of blood vessel images respectively acquired using irradiation light of a wavelength easily absorbed by oxyhemoglobin within an object and irradiation light of a wavelength easily absorbed by deoxyhemoglobin, the oxygen saturation within blood can be measured. The distribution situation of the oxygen saturation is imaged in the oxygen saturation distribution image. This can be expressed, for example, as a color image in which different hues are allocated in accordance with the numerical value of the oxygen saturation.

In this example, a threshold parameter managed by the threshold management module 153 is the light absorption coefficient.

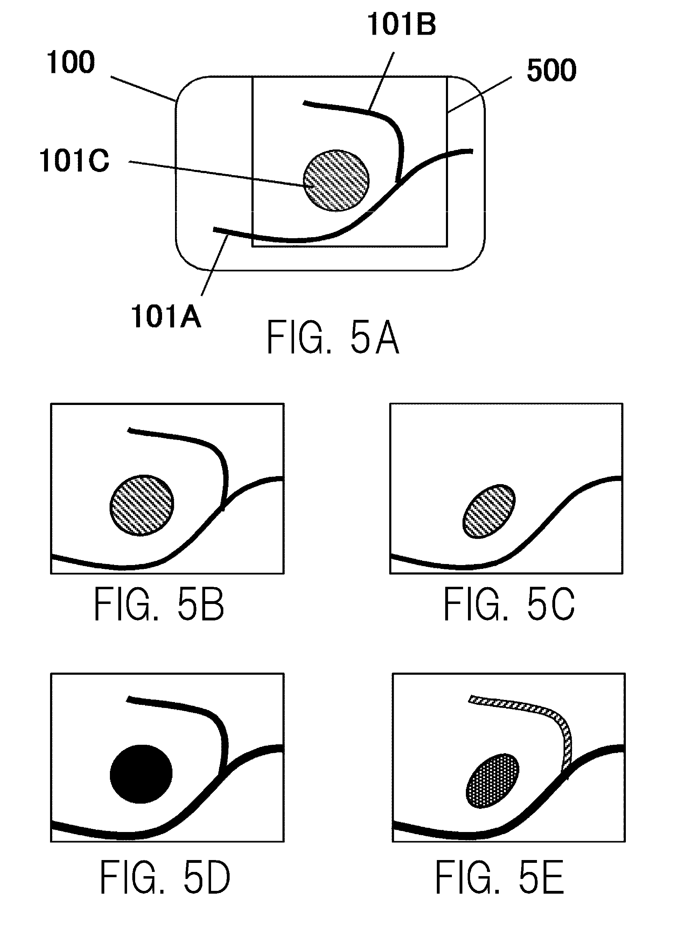

Hereinafter, image processing for object information acquisition in this example will be described with reference to Figs. 5A to 5E. Fig. 5A is a relationship diagram of an object and a region of interest (ROI). In the object 100, a region 500 is the ROI. The object 100 includes light absorbers 101A and 101B that are each a separate blood vessel and a tumor area 101C.

Fig. 5B is a base image determined from a blood vessel image acquired with a specific wavelength out of a plurality of acquired blood vessel images. The threshold parameter (light absorption coefficient in this example) is applied with respect to the base image, and a portion of a predetermined value or greater is extracted. The result is a region segmentation of the base image into a segment under attention and a segment not under attention, as shown in Fig. 5C. The light absorber 101A that is a blood vessel of a large scale is included in the segment under attention, but the light absorber 101B is in the segment not under attention since the scale is small and the light absorption coefficient is small.

Fig. 5D is a distribution image of oxygen saturation obtained by measurement with two wavelengths. As can been seen from the drawing, an image of the light absorber 101A that is the main blood vessel is included, but the visibility of the blood vessel is reduced by an image derived from a photoacoustic wave from a surrounding tissue. The light absorber 101B and a surrounding tissue are also imaged. If this functional image is superimposed on a morphological image without any processing, there is a risk of affecting diagnosis.

The parameter for image processing in this example is the transmittance. That is, for a region of a segment not under attention out of respective regions divided as in Fig. 5C, the transmittance of an oxygen saturation image to be superimposed is increased, so that an ultrasound wave image on which superimposition is performed is made more visible. Fig. 5E is a superimposition functional image obtained in this example. This is obtained by performing transparent image processing with respect to the oxygen saturation distribution image on the basis of the region divided in a manner described above. It can be seen that the light absorber 101B in the segment not under attention is high in transmittance.

The processing described above is almost tantamount to performing mask processing with respect to the oxygen saturation distribution image depending on the scale of blood vessel image under attention. At this time, which one of artery that is high in oxyhemoglobin and vein that is high in deoxyhemoglobin contributes more in the blood vessel image can be determined in accordance with the wavelength selected for the base image. For example, it is conceivable to selectively mask arterial blood, so that a new blood vessel is brought to attention. Through superimposition of a blood vessel image created with each wavelength, a blood vessel of a large scale can be extracted regardless of type to display the oxygen saturation.

Whit this example, the oxygen saturation distribution image in which information other than a blood vessel portion (segment under attention) of a predetermined scale or greater is made less conspicuous is superimposed with respect to the morphological image obtained in ultrasound wave measurement. Therefore, the relevance of the oxygen saturation in the segment under attention and the morphological information is checked easily.

### <Example 3>

In this example, one example of a threshold parameter and transparency processing will be described. In Examples 1 and 2, the threshold parameter has been described to be one value. However, in this example, a plurality of parameters are used.

With one threshold parameter, a region greater than or equal to a threshold and a region smaller than the threshold are distinguished in a light absorption image. With a plurality of parameters as in this example, a region (region under attention) in which a blood vessel of a specific scale is present and a region (region not under attention) that is otherwise can be distinguished in more detail.

At this time, the parameter for image processing performed for each of the divided regions is the transmittance. For example, in the case where low transmittance is set for a region under attention and high transmittance for a region not under attention, a superimposition image in which the region not under attention is transparent and the region under attention stands out is created. When a functional image created in this manner is superimposed on a morphological image, a segment necessary for diagnosis can be presented with high visibility.

With this example, a region to be displayed can be designated more precisely in a functional image. By setting the transmittance, a method for a less conspicuous display is made possible, instead of completely eliminating a region not under attention in a functional image from a superimposed image. Thus, there are more options for a user.

As described in each example above, image processing is performed such that a portion not under attention in functional information is made less conspicuous in accordance with a user-designated parameter, upon superimposition and display of a functional image obtained by photoacoustic measurement and a morphological image obtained by ultrasound wave measurement in the present invention. Since a portion under attention in morphological information can be prevented from being covered and hidden by an unnecessary portion in the functional information as a result, comparison of the functional information and the morphological information is easy.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

Provided is an object information acquiring apparatus including a light source, a photoacoustic probe that receives a photoacoustic wave generated at an object irradiated with light, an ultrasound probe that receives an ultrasound wave transmitted to the object and then reflected within the object, and a signal processor that creates a functional image showing functional information within the object on the basis of the photoacoustic wave and creates a morphological image showing morphological information within the object on the basis of the ultrasound wave, the signal processor being configured to divide the functional image into a plurality of regions, perform different image processing for each of the regions, superimpose the processed functional image on the morphological image, and display the resulting image in a display.

## Claims

1. An object information acquiring apparatus comprising:
a light source;
a photoacoustic probe configured to receive a photoacoustic wave generated at an object irradiated with light from the light source;
an ultrasound probe configured to receive an ultrasound wave transmitted to the object and then reflected within the object; and
a signal processor configured to create a functional image showing functional information within the object on the basis of the photoacoustic wave and create a morphological image showing morphological information within the object on the basis of the ultrasound wave,
wherein the signal processor divides the functional image into a plurality of regions, performs different image processing for each of the regions, superimposes the processed functional image on the morphological image, and displays the resulting image in a display.

2. The object information acquiring apparatus according to claim 1, wherein the signal processor performs the image processing in which a different transmittance is set for the plurality of regions of the functional image respectively.

3. The object information acquiring apparatus according to claim 1 or 2, wherein the signal processor performs the image processing in which a different hue is set for the plurality of regions of the functional image respectively.

4. The object information acquiring apparatus according to any one of claims 1 to 3, wherein the light source irradiates light of a plurality of wavelengths, and
the signal processor creates, as the functional image, an oxygen saturation distribution image within the object on the basis of a photoacoustic wave corresponding to each of the plurality of wavelengths, performs different image processing on the oxygen saturation distribution image for each of the regions, superimposes the processed oxygen saturation distribution image on the morphological image, and displays the resulting image.

5. The object information acquiring apparatus according to any one of claims claim 1 to 4, wherein the photoacoustic probe also acts as the ultrasound probe.

6. The object information acquiring apparatus according to any one of claims 1 to 5, wherein the ultrasound wave transmitted to the object is an ultrasound wave transmitted from the ultrasound probe.

7. The object information acquiring apparatus according to any one of claims 1 to 6, wherein the signal processor divides the functional information into a plurality of regions on the basis of a predetermined threshold.

8. The object information acquiring apparatus according to claim 7, wherein the signal processor creates the functional image showing a light absorption coefficient as the functional information and reduces a transmittance of a region in which the functional information is greater than or equal to the predetermined threshold.

9. The object information acquiring apparatus according to claim 7 or 8, further comprising an input unit for a user to indicate the predetermined threshold.

10. The object information acquiring apparatus according to any one of claims 1 to 6, further comprising an input unit for a user to indicate a boundary of the plurality of regions.
